# EUROPEAN PATENT SPECIFICATION

(11) **EP 4 265 282 B1**
(45) Date of publication and mention of the grant of the patent: **25.02.2026**
(21) Application number: 21910259.7
(22) Date of filing: 06.12.2021
(51) Int. Cl.: A61M 1/16, A61M 1/36, G01L 19/00

(54) **PRESSURE MEASURING DEVICE AND MEDICAL DEVICE**
DRUCKMESSVORRICHTUNG UND MEDIZINISCHE VORRICHTUNG
DISPOSITIF DE MESURE DE PRESSION ET DISPOSITIF MÉDICAL

(30) Priority: 21.12.2020 JP 2020211632
(43) Date of publication of application: 25.10.2023
(73) Proprietor: Nikkiso Co., Ltd., Tokyo 150-6022 (JP)
(72) Inventor: MOCHIZUKI, Hiroaki, Makinohara-shi, Shizuoka 421-0496 (JP); SUZUKI, Tomohiro, Makinohara-shi, Shizuoka 421-0496 (JP); HASEGAWA, Shinya, Makinohara-shi, Shizuoka 421-0496 (JP)
(74) Representative: von Hirschhausen, Helge
(86) International application number: PCT/JP2021/044720
(87) International publication number: WO 2022/138109

(56) References cited:
- WO-A1-2020/250839
- JP-A- 2017 504 389
- JP-A- 2019 124 636
- JP-A- 2020 089 588
- JP-A- 2020 089 588
- US-A1- 2016 228 631
- US-A1- 2020 338 254

## Description

### Technical Field

The present invention relates to a pressure measuring device and a medical device, in which a pressure sensor is detachably connected to a space where pressure fluctuates.

### Background Art

As a medical device, a pressure measuring device is disposed at a location along a fluid flow channel to detect and measure pressure in a space where pressure fluctuates in accordance with a fluid flowing in the fluid flow channel, so as to, e.g., adjust the flow of the fluid. For example, in a device for performing a purification treatment on blood, i.e., a dialysis treatment, the flow of blood in a blood circuit in which the blood circulates outside the body is detected by a pressure sensor and adjusted (see, for example, PTL 1).

In this blood purification apparatus (fluid flowing apparatus), because the blood circuit needs to be replaced for every treatment for safety enhancement, the pressure measuring device is fabricated and structured to be detachably connectable to the blood circuit.

### Citation List

### Patent Literature

PTL 1: Japanese Patent Laid-Open No. 2020-89588

### Summary of Invention

### Technical Problem

Because such a pressure measuring device detects pressure fluctuations in order to adjust the flow of a fluid, pressure needs to be measured with high accuracy and with high quality. However, should a foreign matter such as a fiber be stuck in a connection location that enables attachability and detachability to and from the pressure fluctuating space side, leakage may occur, which can lead to problems such as not being able to adjust fluid flow with high reliability.

Thus, the present invention has an object to enable highly reliable fluid flow adjustment by ensuring the quality of connection of a pressure measuring device to achieve highly accurate pressure measurement.

### Solution to Problem

An aspect of the present invention for achieving the above object is a pressure measuring device to and from which a detaching-attaching member including a fluid flow channel in which a fluid is flowable is attached and detached and which measures pressure in a measurement target which is the attaching-detaching member, the pressure measuring device including: a communication flow channel placed to allow communication of gas with the fluid flow channel; a connection portion to which a connection counterpart portion of the attaching-detaching member is connected to bring the fluid flow channel and the communication flow channel to a communication state or a non-communication state; a pressure sensor that measures pressure in the fluid flow channel and the communication flow channel in a case where the connection counterpart portion is connected to the connection portion to bring the fluid flow channel and the communication flow channel in the communication state; a pressure increasing-decreasing unit that increases or decreases pressure in the communication flow channel to discharge or suction gas outside; and a driving unit that causes the pressure increasing-decreasing unit to be driven manually or automatically at a timing at which a first contact surface of the connection portion to be brought into airtight contact with the connection counterpart portion is away from the connection counterpart portion, in which the first contact surface of the connection portion is formed at a location where gas is discharged or a location where gas is suctioned by the pressure increasing-decreasing unit increasing or decreasing the pressure in the non-communication state where the connection portion is away from the connection counterpart portion.

An aspect of the invention of a medical device for solving the above problem includes the pressure measuring device described above, the attaching-detaching member includes, as the fluid flow channel, a blood flow channel in which blood is flowable, the attaching-detaching member includes, as part of the blood flow channel, a chamber capable of retaining blood and gas, and the measurement target is an internal space of the chamber.

### Advantageous Effects of Invention

Thus, according to aspects of the present invention, a pressure measuring device can be connected to a pressure fluctuating space with high quality and thus can measure pressure with high accuracy. Thus, what is aimed to achieve is, e.g., highly reliable fluid flow adjustment.

### Brief Description of Drawings

[Fig. 1] Fig. 1 is a perspective view showing the outer appearance of a blood purification apparatus as an example of a medical device including a pressure measuring device according to an embodiment of the present invention.
[Fig. 2] Fig. 2 is a circuit diagram schematically showing a route that blood, which is a fluid, flows.
[Fig. 3] Fig. 3 is a perspective view showing the main body side and a cover of a blood pump.
[Fig. 4] Fig. 4(a) is a perspective view showing a circuit chamber that forms part of a blood circuit and a joint that enables connection to the pressure measuring device, and Fig. 4(b) is a perspective view showing the joint.
[Fig. 5] Fig. 5(a) is a longitudinal sectional view showing the circuit chamber that forms part of the blood circuit, Fig. 5(b) is a longitudinal sectional view showing the joint that enables connection of the circuit chamber to the pressure measuring device.
[Fig. 6] Fig. 6 is a longitudinal sectional view showing a state where the joint is connected to the circuit chamber shown in Fig. 5.
[Fig. 7] Fig. 7(a) is a longitudinal sectional view of a main part, illustrating a situation where the circuit chamber shown in Fig. 5 is about to be connected to the joint, and Fig. 7(b) is a longitudinal sectional view of the main part, illustrating a situation where the circuit chamber is being removed from the joint.
[Fig. 8] Fig. 8 is a block diagram illustrating the connection configuration of a controller that controls the entire device.
[Fig. 9] Fig. 9 is a schematic diagram showing the configuration of a main part during the work of connecting the blood circuit.
[Fig. 10] Fig. 10 is a schematic diagram showing the configuration of the main part of a different circuit chamber on a blood circuit.
[Fig. 11] Fig. 11 is a diagram showing a different configuration for connecting the joint to the circuit chamber, Fig. 11(a) being a sectional view showing a state during a connection preparation, Fig. 11(b) being a sectional view showing a state during a connection action.

### Description of Embodiments

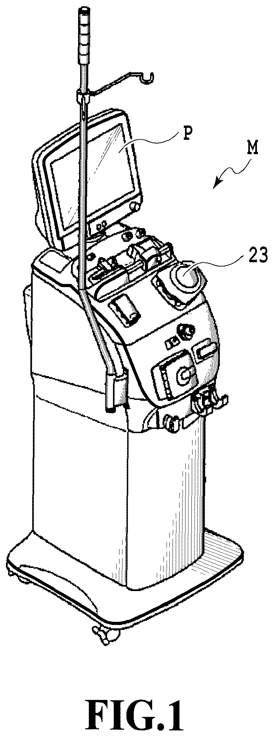

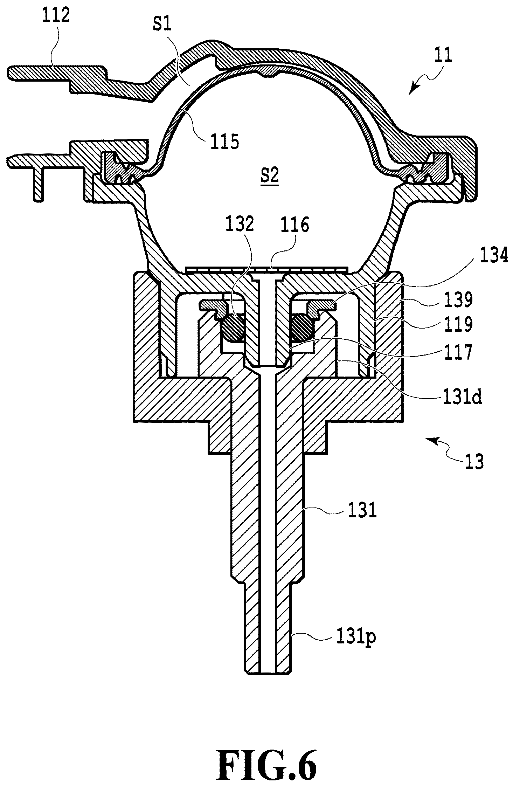

With reference to the drawings, embodiments of the present invention are described in detail below. Figs. 1 to 9 are diagrams showing a blood purification apparatus which is an example of a medical device that includes a pressure measuring device according to a first embodiment of the present invention.

### <First Embodiment>

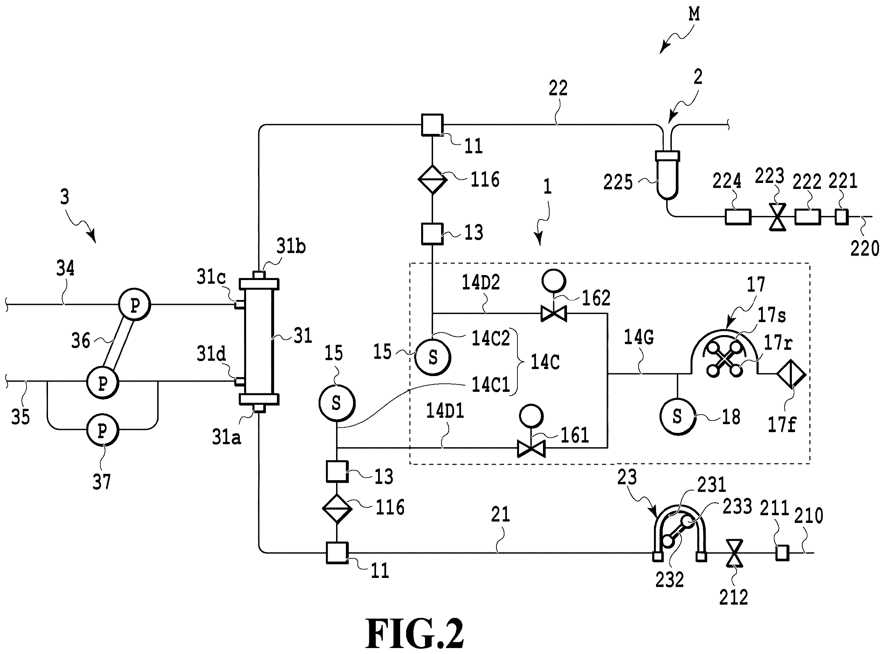

In Figs. 1 and 2, a blood purification apparatus M includes two pressure measuring devices 1, a blood circuit 2, and a dialysis circuit 3, and is built as a medical device for performing a dialysis treatment (blood purification treatment) on a patient by driving the components of the apparatus including the blood circuit 2 and the dialysis circuit 3 based on measurement results from each of the pressure measuring devices 1 in response to an input operation performed on an operation panel (an operation unit) P. The blood circuit 2 includes an arterial-side blood circuit 21 and a venous-side blood circuit 22 and circulates the blood of a patient using a blood pump 23 so that the blood may pass through a dialyzer 31. The dialysis circuit 3 includes the dialyzer 31 that connects the arterial-side blood circuit 21 and the venous-side blood circuit 22 of the blood circuit 2 and a dialysate introduction line 34 and a dialysate drainage line 35 that are connected to the dialyzer 31, and in addition to these two system, a dual pump 36, and causes the dialysate in these lines 34, 35 to flow. This blood purification apparatus M is fabricated to have a structure that undetachably includes the pressure measuring devices 1 along with the dialysate introduction line 34, the dialysate drainage line 35, and the dual pump 36 of the dialysis circuit 3, and is built such that the arterial-side blood circuit 21 and the venous-side blood circuit 22 of the blood circuit 2 as well as the dialyzer 31 of the dialysis circuit 3 are detachably attached and used disposably.

Here, the dialyzer 31 has the dialysate introduction line 34 connected to its dialysate introduction inlet 31c and the dialysate drainage line 35 connected to its dialysate introduction outlet 31d and thereby forms a flow channel in which a dialysate flows through hollow fibers (not shown) thereinside. The dialyzer 31 performs a dialysis treatment while passing the blood of a patient flowing in the blood circuit 2 by utilizing the blood purification membranes of the hollow fibers. The dual pump 36 is disposed to extend over both of the dialysate introduction line 34 and the dialysate drainage line 35 and is driven to pump the dialysate in both of an introduction direction and a drainage direction so that the dialysate may pass the dialyzer 31 stably. Note that the dialysate introduction line 34 is connected to a dialysate supply device (not shown) at its one end side and supplies a dialysate to the dialyzer 31 via the dialysate introduction inlet 31c at the other end side while adjusting the density of the dialysate, and the dialysate drainage line 35 is connected to drainage means (not shown) at its one end side and sends the dialysate which has been used for the dialysate treatment out via the dialysate introduction outlet 31d at the other end side. Also, a water removal pump 37 is disposed on the dialysate drainage line 35 in such a manner as to bypass the dual pump 36, so as to remove water from the blood of the patent flowing through the dialyzer 31.

The arterial-side blood circuit 21 of the blood circuit 2 has its one end side connecting to and communicating with a blood introduction inlet 31a of the dialyzer 31 of the dialysis circuit 3 and has an arterial-side puncture needle 210 at the other end side inserted into and communicating with a blood vessel of a patient, so as to receive the blood of the patient to be dialyzed by the dialyzer 31 in a treatable manner (i.e., blood removal). This arterial-side blood circuit 21 has the pressure measuring device 1 and the blood pump 23 at locations along the circuit. The pressure measuring device 1 measures pressure which is in accordance with the flow of blood in the circuit, and the blood pump 23 pumps the blood so that the blood can flow stably in the circuit. Note that the following are disposed on the arterial-side blood circuit 21: a connector 211 for linking and connecting the arterial-side puncture needle 210 and a clamp 212 for adjusting and controlling the flow of blood in the circuit by opening and closing of a valve.

Here, the blood circuit 2 (21, 22) is formed of an elastic deformable tube that houses blood, which is a fluid, in a fluid flow channel thereinside so that the blood can flow therein. Meanwhile, the blood pump 23 is configured as what is called a tube pump (roller pump), which forwardly and reversely moves a location to compress the tubular arterial-side blood circuit 21 of the blood circuit 2 and thereby pumps and sends the blood in the fluid flow channel to the downstream side. Upstream fluid is drawn in continuously by the negative pressure generated by the pumping of the fluid to the downstream side.

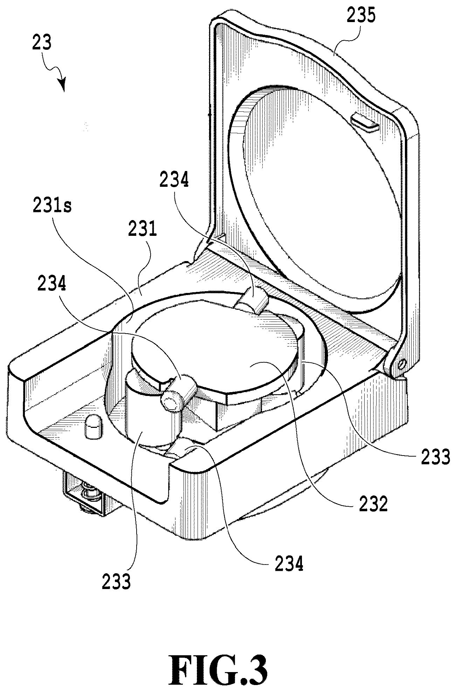

The blood pump 23 is, as shown in Fig. 3, fabricated to have a structure such that a roughly disk-shaped rotor 232 is rotatably housed in a circular, concave-shaped stator 231, and the tubular arterial-side blood circuit 21 is set by being wound around the circular outer circumferential side of the rotor 232 and sandwiched between the circular outer circumferential side of the rotor 232 and a circular inner wall 231s of the stator 231. In this blood pump 23, a pair of rollers 233 are rotatably disposed in parallel to the rotary axis, at symmetric positions opposite from each other in a direction orthogonal to the axis of the disk-shaped rotor 232 (opposite from each other in a diameter direction). The blood pump 23 is configured such that the pair of rollers 233 are driven and rotated by following the rotational driving of the rotor 232 while pressing the arterial-side blood circuit 21 against the circular inner wall 231s of the stator 231 and compress and close the inner surface of the tube in a liquid-tight manner. The blood pump 23 can thus pump the blood in the tube of the arterial-side blood circuit 21 to the downstream side by rotating the rotor 232, and in a state of stopping the rotation of the rotor 232, can stop the flow of blood in the tube by the rollers 233 compressing the arterial-side blood circuit 21 against the circular inner wall 231s of the stator 231 in a liquid-tight manner.

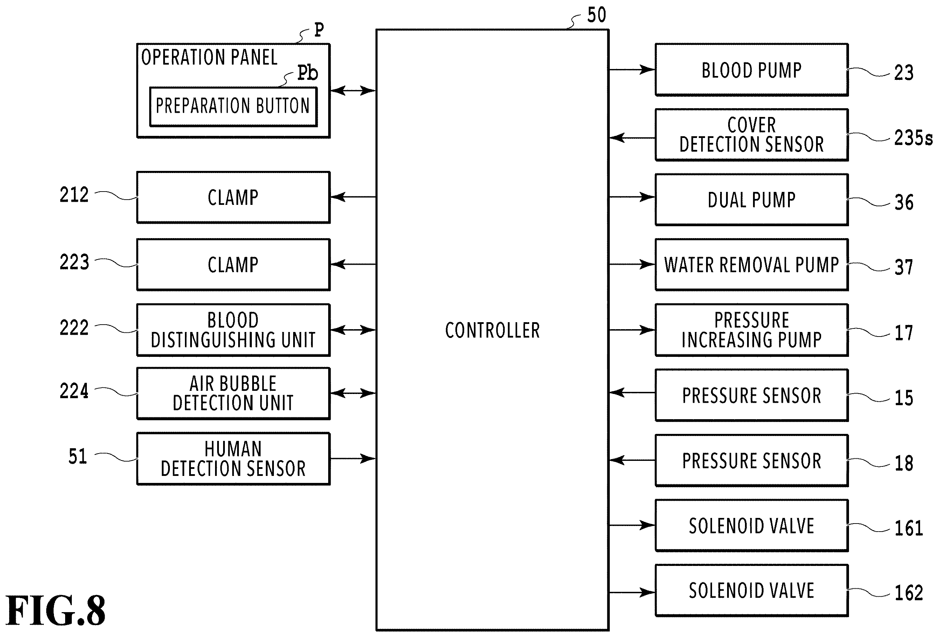

Also, the blood pump 23 includes a cover 235 that covers the outer surface of the stator 231 to restrict interference with, e.g., the rotor 232 that is rotating and the arterial-side blood circuit 21 that is set therein, and the blood pump 23 has a cover detection sensor 235s (shown in Fig. 8) that detects opening and closing of the cover 235 to prevent accidents from occurring. This blood pump 23 is configured such that guides 234 extending in a direction orthogonal to the axis of the disk-shaped rotor 232 (in the diametrical direction) are placed at upper and lower four locations in total near the pair of rollers 233 to help prevent the tubular arterial-side blood circuit 21 wrapped around the rotor 232 from coming out of the stator 231.

The venous-side blood circuit 22 of the blood circuit 2 has its one end side connecting to and communicating with a blood introduction outlet 31b of the dialyzer 31 of the dialysis circuit 3 and has a venous-side puncture needle 220 at the other end side inserted into and communicating with a blood vessel of the patient and is thus configured to return the blood that has undergone the dialysis treatment by the dialyzer 31 to the patient end (i.e., blood return). This venous-side blood circuit 22 has the pressure measuring device 1 and a vein chamber 225 placed at locations along the circuit. The pressure measuring device 1 measures pressure which is in accordance with the flow of the blood in the circuit, and the vein chamber 225 separates air bubbles present in the blood on the venous-side blood circuit 22 side caused to flow (pumped) by the blood pump 23 and safely returns normal blood to the patient. Note that the following are disposed on the venous-side blood circuit 22: a connector 221 for linking and connecting the venous-side puncture needle 220, a blood distinguishing unit 222 that distinguishes whether the fluid flowing in the circuit is blood, a clamp 223 that adjusts and controls the flow of blood in the circuit by opening and closing of a valve, and an air bubble detection unit 224 that detects air bubbles present in the blood flowing in the circuit. It goes without saying that the blood distinguishing unit 222 may be provided on the arterial-side blood circuit 21 side or on both of the blood circuits 21, 22.

Then, the two pressure measuring device 1 are placed separately for the arterial-side blood circuit 21 and the venous-side blood circuit 22 of the blood circuit 2, and enable the blood of the patient to be pumped (caused to flow) stably at optimal pressure at the arterial side and at the venous side by detecting and measuring pressure that fluctuates according to the flow of the blood of the patient inside the blood circuit 2, as will be described later.

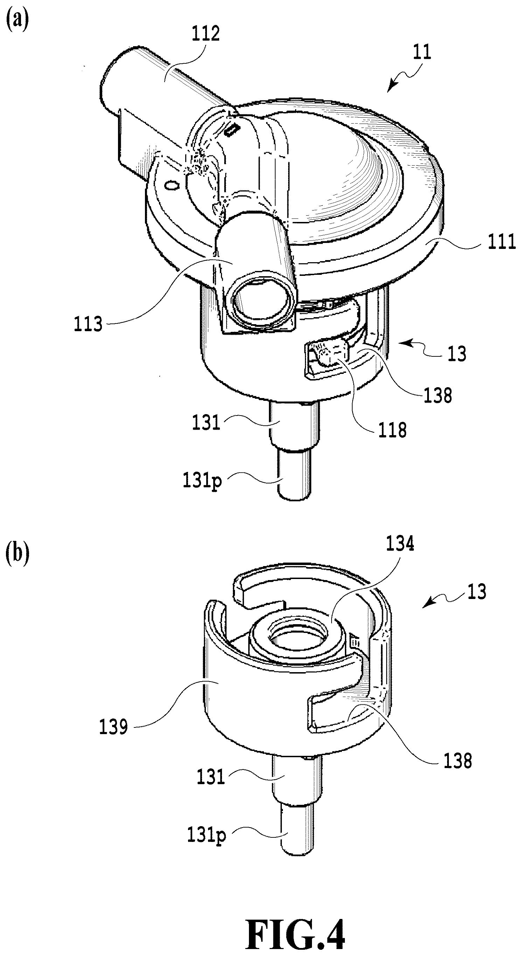

As shown in Figs. 4 to 6, the pressure measuring devices 1 are each built such that a pressure sensor 15 is connected by detachably attaching a joint 13 to a circuit chamber (attaching-detaching member) 11 in order to measure the pressure in the circuit chamber 11, the circuit chamber 11 being detachably placed to be located at a location along the flow channel of a corresponding one of the arterial-side blood circuit 21 and the venous-side blood circuit 22 of the blood circuit 2. Note that although Fig. 2 shows a schematic diagram where the circuit chamber 11 and the joint 13 are linked with a distance from each other, they are fabricated to have attachable/detachable structures to form an integral body with a filter 116 in between, as will be described later. By connecting a connection tube 14C to a cylindrical protrusion 131p to be described later, the joint 13 allows the space pressure in the circuit chamber 11 to reach the pressure sensor 15, so that the pressure fluctuated by the flow of blood in the blood circuit 2 can be detected (measured).

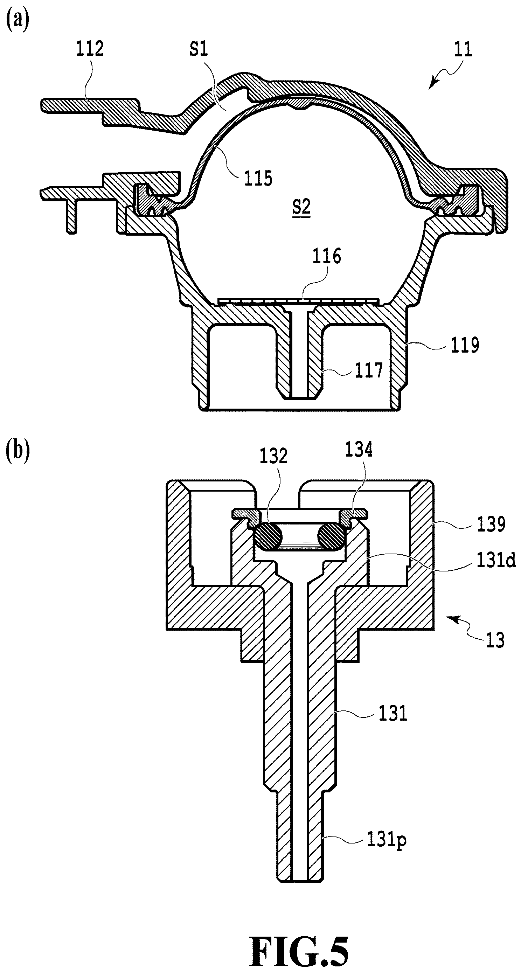

As shown in Fig. 4(a) and Fig. 5(a), the circuit chambers 11 each include connection ports 112, 113 communicating with a space S formed in a housing 111 and form part of the fluid flow channel which runs continuously via the dialyzer 31 by connecting the connection ports 112, 113 so that the space S may be interposed at locations along the blood circuit 2 (21, 22).

This circuit chamber 11 is fabricated to bisect the space S into a communication space S1 and a pressure space S2 by placing a diaphragm 115 in the housing 111. The communication space S1 functions as a fluid flow channel where the connection ports 112, 113 communicate with each other (a blood flow channel in which blood flows), and the pressure space S2 functions such that its volume fluctuates as the diaphragm 115 deforms in accordance with the flow of blood in the communication space S1.

Also, the circuit chamber 11 includes an output port 117 communicating with the pressure space S2 via the filter 116. This circuit chamber 11 is fabricated to have an integral structure by insertion, linkage, and fixation of a hollow-cylindrical insertion coupling 119 formed around the output port 117 into a hollow-cylindrical reception coupling 139 of the joint 13 to be described later, so that the output port (connection counterpart portion) 117 is linked to a coupling flow channel (connection portion) 131 of the joint 13. Note that the filter 116 of the circuit chamber 11 prevents blood from flowing out through the output port 117 into the joint 13 on the pressure sensor 15 side in case the diaphragm 115 breaks or the like to cause blood in the communication space S1 to flow into the pressure space S2.

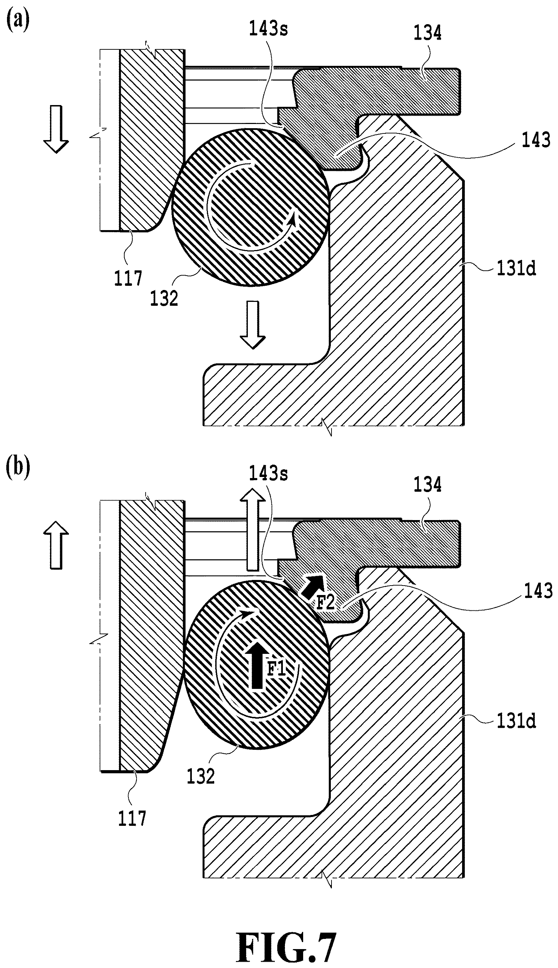

As shown in Figs. 4 to 6, the joint 13 is fabricated to establish airtight linkage and connection such that with an O-ring 132 made of an elastic material being housed inside a large diameter portion 131d formed at one end side of the coupling flow channel (connection portion) 131, (a first contact surface of) the O-ring 132 is sandwiched in a close contact between the large diameter portion 131d and the outer surface of the output port (connection counterpart portion) 117 of the circuit chamber 11 to be inserted thereinto (see Fig. 7(a)). Also, the joint 13 has a cap 134 placed at an end surface of the large diameter portion 131d, which is at the one end side of the coupling flow channel 131, the cap 134 being made of an elastic material formed into a flat ring shape. A short flange 143 is formed at the ring-shaped rim of the cap 134, the flange 143 having an inner circumferential slant surface 143s located at the inner circumferential side of the large diameter portion 131d of the coupling flow channel 131.

As shown in Fig. 7(b), this joint 13 is fabricated to make it difficult for the circuit chamber 1 1 to come off because in an event where the insertion coupling 119 of the circuit chamber 11 is pulled away from the coupling flow channel 131, the O-ring 132, which deforms between them and moves in a direction of arrow F1, abuts against the inner circumferential slant surface 143s of the cap 134 and presses the flange 143 against the inner surface of the large diameter portion 131d in a direction indicated by arrow F2. Also, the cap 134 can function as a retainer for preventing the O-ring 132 from coming off from the large diameter portion 131d in an event where the insertion coupling 119 of the circuit chamber 11 is pulled out from the coupling flow channel 131.

Here, the hollow-cylindrical reception coupling 139 of the joint 13 into which to insert and fit the insertion coupling 119 of the circuit chamber 11 is fixed and attached to the one end side of the coupling flow channel 131, outside of the large diameter portion 131d, and the cylindrical protrusion 131p to which to connect the connection tube 14C that communicates with the pressure sensor 15 is formed at the other end side of the coupling flow channel 131. By becoming integral with the circuit chamber 11, the joint 13 links the output port 117 of the circuit chamber 11 to the coupling flow channel 131 and thereby enables the circuit chamber 11 to connect to and communicate with the pressure sensor 15 via the connection tube (communicating flow channel) 14C.

Also, an engagement protrusion 118 is formed on the outer surface of the hollow-cylindrical insertion coupling 119 of the circuit chamber 11, and a letter-L-shaped crank groove 138 is formed at the joint 13, extending continuous from an opening-side edge of the hollow-cylindrical reception coupling 139. In an event where the insertion coupling 119 is inserted into the reception coupling 139, the engagement protrusion 118 is inserted into the crank groove 138 and relatively rotated at a fitting position, and in this way, the circuit chamber 11 and the joint 13 can be locked so that simple movement in a direction of the axis of the hollow-cylindrical shapes does not allow pulling off.

Also, in the pressure measuring devices 1 of the present embodiment, a pair of branching tubes 14D1, 14D2 and a common tube 14G also connect to and communicate with the coupling flow channels 131 of the joints 13 integral with the circuit chambers 11 on the respective circuits of the blood circuit 2 (21, 22), in addition to the connection tubes 14C1, 14C2 connecting to and communicating with them. To be more specific, the one end sides of the pair of branching tubes 14D1, 14D2 connect to, communicate with, and branch off from the pair of connection tubes 14C1, 14C2 that connect to and communicate with the coupling flow channels 131 of the joints 13, and the other end sides of the branching tubes 14D1, 14D2 merge and are gathered, connecting to and communicating with the one end side of the common tube 14G.

The branching tube 14D1 branches off from the connection tube 14C1 through which the pressure sensor 15, in a functionable manner, connects to and communicates with the joint 13 integral with the circuit chamber 11 on the arterial-side blood circuit 21 side, and has a solenoid valve (an opening-closing unit) 161 placed at a location before the common tube 14G, the solenoid valve 161 opening and closing the communication flow channel. Similarly, the branching tube 14D2 branches off from the connection tube 14C where the pressure sensor 15 on the venous-side blood circuit 22 is placed, and has a solenoid valve 162 placed at a location before the common tube 14G, the solenoid valve 162 opening and closing the communication flow channel.

The common tube 14G has a pressure increasing pump (pressure increasing-decreasing unit) 17 disposed (at its other end side) at a side opposite from the branching tubes 14D1, 14D2 that are at its one end side and a pressure sensor 18 placed at a location along the flow channel in a functionable manner, the pressure increasing pump 17 introducing increased pressure air toward the joints 13 via the connection tubes 14C1, 14C2. This pressure increasing pump 17 includes a filter 17f for preventing contamination of introduced air by dust or the like. The pressure increasing pump 17 here has a function to be able to increase and decrease the pressure on the joint 13 side by driving both in the normal rotation and the reverse rotation, but is not limited to this. The pressure increasing pump 17 may be a dedicated device with a capability of rotating only in the normal direction (increasing pressure) or in the reverse direction (decreasing pressure). For example, in a case of driving for decreasing pressure, a fluid (air) to be caused to flow by suction operation can be blown onto a desired location, as is similar to increased pressure air to be described later.

The pressure increasing pump 17 here is configured of a tube pump having a capability similar to the blood pump 23, and is configured to send increased pressure air toward the joints 13 of the blood circuit 2 (21, 22) via the branching tubes 14D1, 14D2 and the connection tubes 14C1, 14C2 by making rotor-side rollers 17r rotate with the common tube 14G being sandwiched and compressed between the rotor-side rollers 17r and a stator 17s. Compared to the blood pump 23 in which the rotor-side rollers 233 are disposed at two locations in a diametrical direction at equal intervals, this pressure increasing pump 17 has the rotor-side rollers 17r disposed at four locations at equal intervals to compress the common tube 14G against the stator 17s in an airtight manner. Thus, the pressure increasing pump 17 pumps the air in the common tube 14G toward the branching tubes 14D1, 14D2 by rotating the rotor-side rollers 17r, and in a state where the rollers 17r are stopped from rotating, the pressure increasing pump 17 can close the common tube 14G to inhibit flowing therethrough, without provision of a valve.

Incidentally, the blood purification apparatus M executes a dialytic treatment, which is a purification treatment on the blood of a patient, by causing a controller 50 shown in Fig. 8 to perform overall control of the components of the apparatus, including the blood pump 23, the dual pump 36, and the like, so that the blood of the patient may pass through the dialyzer 31 via the arterial-side blood circuit 21 and the venous-side blood circuit 22, and in tandem with this, a dialysate may pass through the dialyzer 31 via the dialysate introduction line 34 and the dialysate drainage line 35.

The controller 50 is configured including a CPU and various kinds of memory such as RAM and ROM and carries out various kinds of control processing including a dialytic treatment by executing prestored control programs in response to an operation inputted on an operation panel P, based on detection information obtained and parameters stored.

To be more specific, as shown in Fig. 8, the controller 50 has connected thereto the components of the apparatus including the clamps 212, 223, the blood distinguishing unit 222, the air bubble detection unit 224, and the blood pump 23 that are placed on the blood circuit 2, as well as the dual pump 36 and the water removal pump 37 that are placed on the dialysis circuit 3, along with the operation panel P and a human detection sensor 51, in such a manner that control signals and the like can be exchanged therebetween. On the assumption of the existence of a user, such as a nurse, operating the operation panel P, this controller 50 provides a treatment for a patient by performing a dialytic treatment that purifies the blood of the patient by controlling driving of the components of the apparatus based on an input operated on the operation panel P to pass each of the blood of the patient and a dialysate through the dialyzer 31 at a certain flow rate and pressure. Here, the operation panel P includes an operation area and a display area. The operation area is for performing various input operations for the blood purification apparatus M, such as, for example, inputting and setting driving conditions, and the display area is for outputting and informing of driving time and the like. On the operation panel P, a message for prompting various operations, a preparation button Pb for starting and stopping a preparation action to be carried out before starting a dialytic treatment to be described later are disposed so that a user such as a nurse can operate it.

The controller 50 is also connected to the pressure sensors 15, 18, the solenoid valves 161, 162, and the pressure increasing pump 17 in such a manner that various signals and the like can be exchanged therebetween. To perform a dialytic treatment, the controller 50 closes the solenoid valves 161, 162 and has the pressure sensors 15 detect pressures in the pressure spaces (measurement targets) S2 of the circuit chambers 11 communicating therewith via the connection tubes 14C1, 14C2, and obtains pressure fluctuations in the communication spaces S1 of the circuit chambers 11. Thereby, the controller 50 can keep track of the flow quality of the blood of the patient in each of the arterial-side blood circuit 21 and the venous-side blood circuit 22 of the blood circuit 2 and can carry out the dialytic treatment safely and properly by, for example, displaying and outputting various messages on the operation panel P.

In this event, while continuing the dialytic treatment of having the pressure sensors 15 detect the pressures in the pressure spaces S2 of the circuit chambers 11 and obtaining the pressure fluctuations in the communication space S1, the controller 50 also functions as a driver for opening the solenoid valves 161, 162 and driving the pressure increasing pump 17. This controller 50 adjusts and controls the flow amount and speed of the blood of the patient by having the pressure increasing pump 17 increase the pressures in the pressure spaces S2 of the circuit chamber 11 (by pumping outside air thereto) via the branching tubes 14D1, 14D2 and the common tube 14G and thereby changing the fluid amounts (pressures) inside the communication spaces S1. Also, at the same time, the controller 50 has the pressure sensor 18 detect the pressure in the common tube 14G and obtains the effectiveness of the increasing of the pressure in the pressure spaces S2 of the circuit chambers 11 for which the pressure sensors 15 detect pressures, or in other words, the increasing of the pressures in the communication spaces S1 of the circuit chambers 11, and performs adjustment and control so as to optimize the driving of the pressure increasing pump 17. Note that although the present embodiment shows an example where one pressure increasing pump 17 is placed to adjust and control the flow amounts and speeds of the blood of a patient in the communication spaces S1 of the circuit chambers 11, the present invention is not limited to this. It goes without saying that the pressure increasing pump 17 may be placed for each of the arterial-side blood circuit 21 and the venous-side blood circuit 22 of the blood circuit 2 to perform the adjustment and control separately.

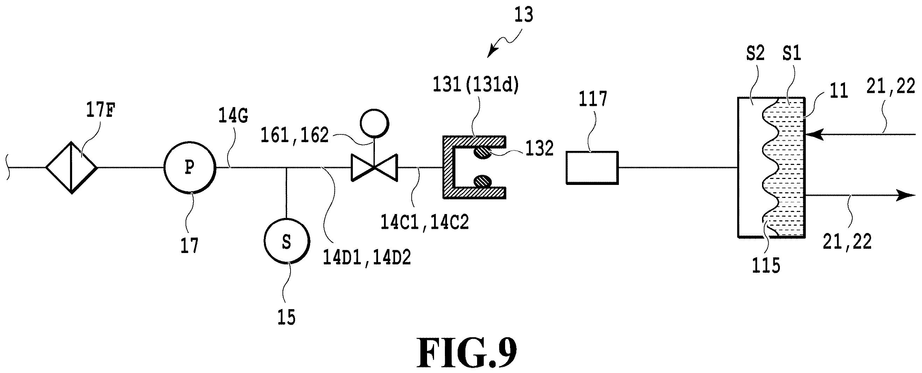

Further, in a case where the preparation button Pb or the like is pressed on the operation panel P in response to a message prompting a preparation action before the circuit chambers 11 are linked to the joints 13 in preparing for a dialytic treatment on the blood flowing in the blood circuit 2 (21, 22), the controller 50 carries out a circuit connection preparation action to be described later to blow off foreign matters attached to the O-rings 132 in the coupling flow channels 131 of the joints 13. To be more specific, for example, as schematically shown in Fig. 9, the controller 50 opens the solenoid valves 161, 162 and drives the pressure increasing pump 17 in response to the preparation button Pb being pressed, to increase the pressure in (pump outside air to) the pressure spaces S2 of the circuit chambers 11 that communicate via the various tubes 14C1, 14C2, 14D1, 14D2, 14G to a desired pressure. After this, in response to the preparation button Pb or the like on the operation panel P being pressed again, the controller 50 closes the solenoid valves 161, 162, stops driving the pressure increasing pump 17, and stands by. The controller 50 can thus carry out the circuit connection preparation action which expels air from the coupling flow channels 131 of the joints 13 before the circuit chambers 11 are linked.

Here, the controller 50 is configured to drive the pressure increasing pump 17 so that the pressure sensor 18 may detect and measure the pressure in the common tube 14G which is increased in pressure enough to blow off foreign matters attached to the O-rings 132 in the coupling flow channels 131 of the joints 13. The pressure sensor 18 is thus different from the pressure sensors 15 that detect and measure pressures in the pressure spaces S2 that fluctuate in accordance with the flow of blood flowing in the communication spaces S1 of the circuit chambers 11, and therefore, the controller 50 is configured to perform the pressure measurement by appropriately adjusting the measurement ranges of the pressure sensors 15, 18.

Thus, the pressure measuring devices 1 are each such that in performing a preparation work for linking the circuit chamber 11 to the joint 13 in order to carry out a dialytic treatment on the blood of a patient flowing in the blood circuit 2, mere pressing of the preparation button Pb on the operation panel P drives the pressure increasing pump 17 to expel air from the coupling flow channel 131 of the joint 13, and then, the insertion coupling 119 can be inserted into the reception coupling 139 to establish airtight linkage.

Thus, the blood purification apparatus M can blow air increased in pressure by the pressure increasing pump 17 onto the O-rings 132 in the large diameter portions 131d of the coupling flow channels 131 of the joints 13 and thereby blow off foreign matters attached thereto.

As a result, the blood purification apparatus M can prevent that pressure fluctuations in accordance with the flow of blood flowing in the communication space S1 of each circuit chamber 11 become unmeasurable because leakage is caused in the pressure space S2 by foreign matters sandwiched between the O-ring 132 in the coupling flow channel 131 of the joint 13 and the outer surface of the output port 117 of the circuit chamber 11.

### <Advantageous Effects of the First Embodiment>

As thus described, in the blood purification apparatus M of the present embodiment, the joints 13 of the pressure measuring devices 1 connect to and communicate with the circuit chambers 11, and the pressure sensors 15 can detect and measure pressure fluctuations in the pressure spaces S2 of the circuit chambers 11 with high accuracy.

Thus, the blood purification apparatus M can adjust the flow of blood in the blood circuit 2 with high accuracy and perform a dialytic treatment on and purify the blood of a patient by causing the blood to flow stably with high reliability.

### <Different Mode of the First Embodiment>

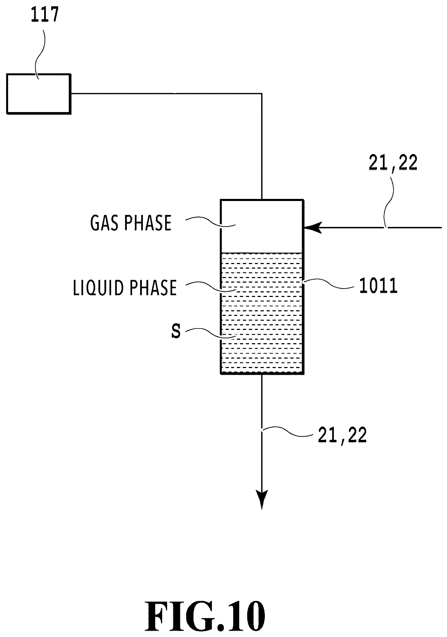

Here, in the example described in the present embodiment, the circuit chamber 11 is of a type (what is called a pressure pod) in which the diaphragm 115, which is a diaphragm, liquid-tightly sections the circuit chamber 11 into the communication space S1, which communicates with the blood circuit 2, and the pressure space S2, which communicates with the coupling flow channel 131 of the joint 13; however, the present invention is not limited to this. For example, as shown in Fig. 10 as a different mode, the present invention can be applied to the blood circuit 2 in which a circuit chamber 1011 of what is called an air trap type is disposed for each of the arterial-side blood circuit 21 and the venous-side blood circuit 22, the circuit chamber 1011 having no diaphragm 115 and including one internal space S.

Specifically, the circuit chamber 1011 is fabricated to form part of the blood circuit 2 in such a mode that the flowing blood flows in from the gas phase side at the upper portion of the internal space S and flows out from the liquid phase side at the lower portion of the internal space S. This circuit chamber 1011 is such that the output port 117 and the insertion coupling 119 are located at the upper portion side of the internal space S and are inserted into the reception coupling 139 of the joint 13 to connect to and communicate with the coupling flow channel 131.

In this different mode, in performing a dialytic treatment, pressure fluctuations in the blood circuit 2 (21, 22) can be obtained by the pressure sensor 15 detecting the pressure in the internal spaces S of the circuit chambers 1011 without the diaphragms 115, and the flow quality of the blood of a patient is kept track so that the dialytic treatment can be carried out safely and properly.

In this event, in this different mode, while the dialytic treatment continues, the pressure sensors 15 detect the pressures in the gas phase in the internal spaces S of the circuit chambers 1011, and the pressure increasing pump 17 is driven in accordance with the pressure fluctuations. Thereby, the pressure in the internal space S is increased (outside air is pumped thereto) to change the liquid surface being the border between the gas phase and the liquid phase, which enables adjustment and control of the flow amount and speed of the blood of a patient.

### <Second Embodiment>

Next, a blood purification apparatus as an example of a medical device including a pressure measuring device according to a second embodiment of the present invention is described. Here, the present embodiment has substantially the same configuration as the above embodiment and therefore uses the same drawings, denotes like configurations using the same reference numerals, and describes characteristic portions (this is also true in the other embodiments to be described later).

As shown in Figs. 1 to 9, the controller 50 of the blood purification apparatus M carries out the circuit connection preparation action in the above embodiment not upon the pressing of the preparation button Pb on the operation panel P like in the above embodiment, but upon detecting, based on a detection signal from the cover detection sensor 235s, that the arterial-side blood circuit 21 has been set by opening and closing the cover 235 of the blood pump 23.

To be more specific, the controller 50 opens the solenoid valves 161, 162 and starts driving the pressure increasing pump 17 at the timing at which the cover 235 of the blood pump 23 is opened and closed and the setting of the arterial-side blood circuit 21 is completed. After that, the controller 50 measures time that has passed since the time of the start of the driving and then, closes the solenoid valves 161, 162 and stops driving the pressure increasing pump 17 at the timing at which it is confirmed that a preset driving time, or for example, a work time long enough to perform the work of connecting the circuit chamber 11 to the joint 13, has passed.

Thus, the pressure measuring devices 1 can ensure that the preparation work for airtightly linking the circuit chambers 11 of the blood circuit 2 to the joints 13 with time to spare is performed with high quality and high reliability, and after this preparation work, the pressure increasing pump 17 is automatically stopped so that the common tube 14G and the like may not be consumed by the squeezing action by the rollers 17r.

### <Advantageous Effects of the Second Embodiment>

In this way, in the blood purification apparatus M of the present embodiment, the pressure increasing pump 17 of the pressure measuring device 1 can reduce load associated with the circuit connection preparation action, and stable driving is enabled by reducing consumption of various components around the pressure increasing pump 17, including the common tube 14G.

### <Third Embodiment>

Next, a blood purification apparatus as an example of a medical device including a pressure measuring device according to a third embodiment of the present invention is described.

As shown in Figs. 1 to 9, the controller 50 of the blood purification apparatus M carries out the circuit connection preparation action in the above embodiment not upon the pressing of the preparation button Pb on the operation panel P like in the above embodiment, but upon detecting, by the human detection sensor 51, a user either operating the operation panel P or setting the arterial-side blood circuit 21 in the blood pump 23.

To be more specific, the controller 50 opens the solenoid valves 161, 162 and starts driving the pressure increasing pump 17 like in the embodiment described above at the timing at which the human detection sensor 51 detects a user in front of the blood purification apparatus M, and closes the solenoid valves 161, 162 and stops driving the pressure increasing pump 17 at the timing at which, for example, it is confirmed that the time measured since the time of the start of the driving exceeds a work time long enough to perform the work of connecting the circuit chambers 11 to the joints 13.

### <Advantageous Effects of the Third Embodiment>

In this way, in the blood purification apparatus M of the present embodiment as well, like in the embodiments described above, the pressure increasing pump 17 of the pressure measuring device 1 can reduce load associated with the circuit connection preparation action, and stable driving is enabled by reduction of consumption of various components around the pressure increasing pump 17, including the common tube 14G.

### <Fourth Embodiment>

Next, a blood purification apparatus as an example of a medical device including a pressure measuring device according to a fourth embodiment of the present invention is described.

As shown in Figs. 1 to 9, in carrying out the circuit connection preparation action of the embodiments described above, the controller 50 of the blood purification apparatus M drives the pressure increasing pump 17 in a shut-off state where the solenoid valves 161, 162 are closed, and after confirming that the increased pressure (measurement value) in the common tube 14G detected by the pressure sensor 18 has reached a preset opening pressure (that the pressure has successfully accumulated), opens the solenoid valves 161, 162 to make a switch to a communication state. After that, the controller 50 closes the solenoid valves 161, 162 and stops driving the pressure increasing pump 17 at the timing at which it is confirmed that the increased pressures in the pressure spaces S2 in the circuit chambers 11 detected by the pressure sensors 15 have reached a preset ending pressure.

Thus, in addition to the effects provided by the embodiments described above, the pressure measuring devices 1 each can effectively blow off foreign matters attached to the O-ring 132 by expelling compressed air retained in the common tube 14G from the coupling flow channel 131 of the joint 13 by driving the pressure increasing pump 17, and also, the pressure measuring device 1 determines completion of linkage for sure and automatically stops the pressure increasing pump 17, thereby enabling reduction in consumption of the common tube 14G and the like by the squeezing action by the rollers 17r.

### <Advantageous Effects of the Fourth Embodiment>

In this way, in addition to the advantageous effects of the embodiments described above, the blood purification apparatus M of the present embodiment can perform, with high quality and high reliability, the work for connecting the output ports 117 of the circuit chambers 11 of the blood circuit 2 to the coupling flow channels 131 of the joints 13 in such a manner as to allow communication therebetween, and stable driving is enabled by reduction of consumption of various components around the pressure increasing pump 17, including the common tube 14G.

### <Fifth Embodiment>

Next, a blood purification apparatus as an example of a medical device including a pressure measuring device according to a fifth embodiment of the present invention is described.

As shown in Figs. 1 to 9, in carrying out the circuit connection preparation action in the embodiment described above, the controller 50 of the blood purification apparatus M opens the solenoid valves 161, 162 and repeats normal rotation (pressure increasing) driving and reverse rotation (pressure decreasing) driving of the pressure increasing pump 17 at certain intervals. After that, the controller 50 closes the solenoid valves 161, 162 and stops driving the pressure increasing pump 17 at the timing at which it is confirmed that a set work time (driving time) has passed since the time of the start of the driving.

Thus, in addition to the effects provided in the embodiments described above, the pressure measuring device 1 can blow compressed air intermittently onto the O-ring 132 in the coupling flow channel 131 of the joint 13 and suction air in the vicinities of the O-ring 132 intermittently by repeating the normal rotation driving and the reverse rotation driving of the pressure increasing pump 17, thereby allowing foreign matters attached to the O-ring 132 to be peeled off effectively.

### <Advantageous Effects of the Fifth Embodiment>

In this way, in addition to the advantageous effects of the embodiments described above, the blood purification apparatus M of the present embodiment can perform, with high quality and high reliability, the work for connecting the output ports 117 of the circuit chambers 11 of the blood circuit 2 to the coupling flow channels 131 of the joints 13 in such a manner as to allow communication therebetween.

### <Sixth Embodiment>

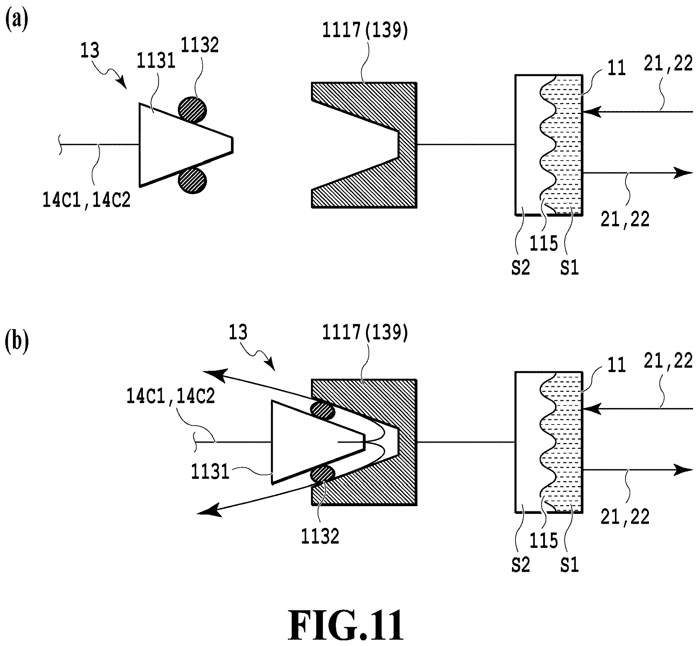

Next, adding Fig. 11, a blood purification apparatus as an example of a medical device including a pressure measuring device according to a sixth embodiment of the present invention is described.

As shown in Figs. 1 to 9, in the blood purification apparatus M, the pressure measuring devices 1 are each made functionable by connecting the joint 13 to the circuit chamber 11 placed at the blood circuit 2 in such a manner as to allow communication therebetween, and the present embodiment, as shown in Fig. 11(a), has a communication structure fabricated such that a coupling flow channel 1131 of the joint 13 is inserted into an output port 1117 communicating with the pressure space S2 in the circuit chamber 11.

Specifically, the coupling flow channel 1131 of the joint 13 is formed in a circular truncated cone shape, and the output port 1117 of the circuit chamber 11 is formed into a coupling shape having an inner surface onto which to fit the circular truncated cone of the coupling flow channel 1131, instead of the reception coupling 139 in the embodiments described above. The coupling flow channel 1131 and the output port 1117 are formed to connect to and communicate with each other airtightly with an O-ring 1132 being sandwiched between the outer surface and the inner surface of their circular truncated cones to be fitted onto each other.

Thus, in carrying out the circuit connection preparation action for linking the circuit chamber 11 to the joint 13 to carry out a dialytic treatment on the blood of a patient flowing in the blood circuit 2, the pressure measuring device 1 can blow increased pressure air from the pressure increasing pump 17 toward the output port 1117 and toward the O-ring 1132 in the coupling flow channel 1131 to blow off foreign matters attached thereto, as shown in Fig. 11(b). In this event, the increased pressure air pumped by the pressure increasing pump 17 is expelled from the coupling flow channel 1131 of the joint 13, and immediately before the linkage to the output port 1117 of the circuit chamber 11, can be blown into and expelled back from the output port 1117, so that foreign matters attached to the O-ring 1132 can be powerfully blown off through a narrow gap between the output port 1117 and the coupling flow channel 1131.

As a result, the blood purification apparatus M can more effectively prevent that pressure fluctuations in accordance with the flow of blood flowing in the communication space S1 of the circuit chamber 11 become unmeasurable because of leakage caused in the pressure spaces S2 by foreign matters sandwiched between the O-ring 132 in the coupling flow channel 1131 of the joint 13 and the output port 1117 of the circuit chamber 11.

### <Advantageous Effects of the Sixth Embodiment>

In this way, the blood purification apparatus M of the present embodiment too can provide advantageous effects similar to those offered by the embodiments described above, and can powerfully blow off foreign matters attached not only to the O-ring 1132 (the first contact surface) but also to the circular truncated cone's inner surface of the output port 1117 (the second contact surface) with which the O-ring 1132 comes into close contact by expelling pressure air through a narrow gap between the output port 1117 of the circuit chamber 11 and the coupling flow channel 1131 of the joint 13.

Thus, the joint 13 of the pressure measuring device 1 can connect to and communicate with the circuit chamber 11 with high quality without any foreign matters stuck therebetween, and thus, pressure fluctuations in the pressure space S2 of the circuit chamber 11 can be detected and measured by the pressure sensor 15 with high accuracy.

As a result, the blood purification apparatus M can adjust the flow of blood in the blood circuit 2 with high accuracy and perform a dialytic treatment on and purify the blood of a patient by causing the blood to flow stably with high reliability.

Although the present embodiment describes a case where with the shapes of the output port 1117 and the coupling flow channel 1131, pressure air from the pressure increasing pump 17 is blown onto the vicinities of the O-ring 1132 immediately before the circuit chamber 11 is linked to the joint 13, and foreign matters attached thereto get stuck, the present invention is not limited to this. For example, a proximity sensor or the like may be disposed for the output port 117 of the circuit chamber 11 and the coupling flow channel 131 of the joint 13, so as to blow pressure air from the pressure increasing pump 17 at the timing at which they approach to an effective distant interval.

Although the medical device described above as an example is a blood purification apparatus (a dialysis apparatus) that causes a fluid which is blood (liquid) to flow, the present invention is not limited to this, and it goes without saying that, for example, the present invention can also be applied to, e.g., a heat-lung machine, which supplies and exhausts a fluid such as breathing air (gas).

Also, although the circuit connection preparation action performed in linking the joint 13 to the circuit chamber 11 is mainly described in the embodiments described above, an action of expelling (or suctioning) air from the coupling flow channel 131 of the joint 13 may be carried out in advance on a regular or irregular basis in addition to or in place of the above action. This can prevent foreign matters from attaching and accumulating at a linkage location on the joint 13 to be linked to the circuit chamber 11 even in a state where the circuit chamber 11 is linked and the blood circuit 2 is unattached. This enables sound maintenance and protection of the status of the apparatus.

### Reference Signs List

- 1: pressure measuring device
- 2: blood circuit
- 3: dialysis circuit
- 11, 1011: circuit chamber
- 13: joint
- 14C, 14C1, 14C2: connection tube
- 14D1, 14D2: branching tube
- 14G: common tube
- 15, 18: pressure sensor
- 17: pressure increasing pump
- 17r, 233: roller
- 17s, 231: stator
- 21: arterial-side blood circuit
- 22: venous-side blood circuit
- 23: blood pump
- 31: dialyzer
- 50: controller
- 51: human detection sensor
- 115: diaphragm
- 116: filter
- 117, 1117: output port
- 119: insertion coupling
- 131, 1131: coupling flow channel
- 132, 1132: O-ring
- 134: cap
- 139: reception coupling
- 143: flange
- 143s: inner circumferential slant surface
- 161, 162: solenoid valve
- 210: arterial-side puncture needle
- 220: venous-side puncture needle
- 235: cover
- 235s: cover detection sensor
- M: blood purification apparatus
- P: operation panel
- Pb: preparation button
- S: internal space
- S1: communication space
- S2: pressure space

## Claims

1. A pressure measuring device (1) to and from which a detaching-attaching member (11) including a fluid flow channel (2) in which a fluid is flowable is attached and detached and which measures pressure in a measurement target which is the attaching-detaching member, the pressure measuring device comprising:
a communication flow channel (14C) placed to allow communication of gas with the fluid flow channel;
a connection portion (131) to which a connection counterpart portion (117) of the attaching-detaching member is connected to bring the fluid flow channel and the communication flow channel to a communication state or a non-communication state;
a pressure sensor (15, 18) that measures pressure in the fluid flow channel and the communication flow channel in a case where the connection counterpart portion is connected to the connection portion to bring the fluid flow channel and the communication flow channel in the communication state;
a pressure increasing-decreasing unit (17) that increases or decreases pressure in the communication flow channel to discharge or suction gas outside; **characterized by**:
a driving unit (50) that causes the pressure increasing-decreasing unit to be driven manually or automatically at a timing at which a first contact surface of the connection portion to be brought into airtight contact with the connection counterpart portion is away from the connection counterpart portion, wherein
the first contact surface (132) of the connection portion is formed at a location where gas is discharged or a location where gas is suctioned by the pressure increasing-decreasing unit increasing or decreasing the pressure in the non-communication state where the connection portion is away from the connection counterpart portion.

2. The pressure measuring device according to claim 1, wherein
the connection portion is fabricated of an elastic material (132) so that the first contact surface is capable of coming into airtight close contact with the connection counterpart portion.

3. The pressure measuring device according to claim 1 or 2, wherein
the communication flow channel includes an opening-closing unit (161, 162) that switches the pressure increasing-decreasing unit and the measurement target to a communicating state or a shut-off state, and
after the pressure in the communication flow channel is increased or decreased in the shut-off state, the opening-closing unit makes a switch to the communicating state, so that gas is blown onto the first contact surface of the connection portion.

4. The pressure measuring device according to any one of claims 1 to 3, wherein
the pressure increasing-decreasing unit has a capability of not only normal-rotation driving to increase pressure in the communication flow channel, but also reverse-rotation driving to decrease pressure in the communication flow channel.

5. A medical device (M) comprising the pressure measuring device according to any one of claims 1 to 4, wherein
the fluid flow channel is a blood flow channel in which blood is flowable,
the attaching-detaching member includes, as part of the blood flow channel, a chamber capable of retaining blood and gas, and
the measurement target is an internal space of the chamber.

6. The medical device according to claim 5, further comprising an operation unit (P) for operating driving of the pressure increasing-decreasing unit.

7. The medical device according to claim 5 or 6, wherein
the driving unit is adapted to automatically start driving of the pressure increasing-decreasing unit once it is confirmed that a user performing operation is detected or operation performed by the user is detected.

8. The medical device according to any one of claims 5 to 7, wherein
the driving unit is adapted to automatically stop driving of the pressure increasing-decreasing unit at a timing at which a value measured by the pressure sensor reaches a preset predetermined pressure.

9. The medical device according to any one of claims 5 to 7, wherein
immediately before the connection portion approaches and is linked to the connection counterpart portion, the pressure increasing-decreasing unit is adapted to discharge or to suction gas between both of the first contact surface of the connection portion and a second contact surface of the connection counterpart portion.

## Patentansprüche

1. Eine Druckmessvorrichtung (1), an der ein Anbring- und Abnehmelement (11) umfassend einen Fluidströmungskanal (2), in dem ein Fluid strömen kann, angebracht und abgenommen wird und die den Druck in einem Messziel, das das Anbring- und Abnehmelement ist, misst, wobei die Druckmessvorrichtung umfasst:
einen Kommunikationsströmungskanal (14C), der so angeordnet ist, dass er eine Kommunikation von Gas mit dem Fluidströmungskanal ermöglicht;
einen Verbindungsabschnitt (131), mit dem ein Gegenverbindungsabschnitt (117) des Anbring- und Abnehmelements verbunden ist, um den Fluidströmungskanal und den Kommunikationsströmungskanal in einen Kommunikationszustand oder einen Nicht-Kommunikationszustand zu versetzen;
einen Drucksensor (15, 18), der den Druck im Fluidströmungskanal und im Kommunikationsströmungskanal misst, wenn der Gegenverbindungsabschnitt mit dem Verbindungsabschnitt verbunden ist, um den Fluidströmungskanal und den Kommunikationsströmungskanal in den Kommunikationszustand zu bringen;
eine Druckerhöhungs- und -verringerungseinheit (17), die den Druck im Kommunikationsströmungskanal erhöht oder verringert, um Gas nach außen abzulassen oder anzusaugen; **gekennzeichnet durch**
eine Antriebseinheit (50), die bewirkt, dass die Druckerhöhungs- und -verringerungseinheit manuell oder automatisch zu einem Zeitpunkt angetrieben wird, zu dem eine erste Kontaktfläche des Verbindungsabschnitts, die in luftdichten Kontakt mit dem Gegenverbindungsabschnitt gebracht werden soll, vom Gegenverbindungsabschnitt entfernt ist, wobei
die erste Kontaktfläche (132) des Verbindungsabschnitts an einer Stelle ausgebildet ist, an der Gas abgelassen wird, oder an einer Stelle, an der Gas durch die Druckerhöhungs- und - verringerungseinheit angesaugt wird, was den Druck im Nicht-Verbindungszustand erhöht oder verringert, in dem der Verbindungsabschnitt vom Gegenverbindungsabschnitt entfernt ist.

2. Die Druckmessvorrichtung nach Anspruch 1, wobei
der Verbindungsabschnitt aus einem elastischen Material (32) hergestellt ist, so dass die erste Kontaktfläche in luftdichten engen Kontakt mit dem Gegenverbindungsabschnitt kommen kann.

3. Die Druckmessvorrichtung nach Anspruch 1 oder 2, wobei
der Kommunikationsströmungskanal eine Öffnungs-Schließeinheit (161, 162) umfasst, die die Druckerhöhungs- und -verringerungseinheit und das Messziel in einen Kommunikationszustand oder einen Sperrzustand schaltet, und
nachdem der Druck im Kommunikationsströmungskanal im Sperrzustand erhöht oder verringert wird, die Öffnungs-Schließeinheit eine Umschaltung in den Kommunikationszustand vornimmt, so dass Gas auf die erste Kontaktfläche des Verbindungsabschnitts geblasen wird.

4. Die Druckmessvorrichtung nach einem der Ansprüche 1 bis 3, wobei
die Druckerhöhungs- und -verringerungseinheit nicht nur einen Normalrotationsantrieb zum Erhöhen des Drucks im Kommunikationsströmungskanal, sondern auch einen Rückwärtsrotationsantrieb zum Verringern des Drucks im Kommunikationsströmungskanal ausführen kann.

5. Ein medizinisches Gerät (M), das die Druckmessvorrichtung gemäß einem der Ansprüche 1 bis 4 umfasst, wobei
der Fluidströmungskanal ein Blutströmungskanal ist, in dem Blut fließen kann,
das Anbring- und Abnehmelement als Teil des Blutströmungskanals eine Kammer umfasst, die Blut und Gas zurückhalten kann, und
das Messziel ein Innenraum der Kammer ist.

6. Medizinisches Gerät gemäß Anspruch 5, das ferner eine Bedieneinheit (P) zum Bedienen des Antriebs der Druckerhöhungs- und -verringerungseinheit umfasst.

7. Medizinisches Gerät nach Anspruch 5 oder 6, wobei
die Antriebseinheit so ausgelegt ist, dass sie den Antrieb der Druckerhöhungs- und - verringerungseinheit automatisch startet, sobald bestätigt wird, dass ein Benutzer, der eine Operation durchführt, oder eine vom Benutzer durchgeführte Operation erkannt wird.

8. Medizinisches Gerät gemäß einem der Ansprüche 5 bis 7, wobei
die Antriebseinheit so ausgelegt ist, dass sie den Antrieb der Druckerhöhungs- und - verringerungseinheit zu einem Zeitpunkt automatisch stoppt, zu dem ein vom Drucksensor gemessener Wert einen voreingestellten vorbestimmten Druck erreicht.

9. Medizinisches Gerät nach einem der Ansprüche 5 bis 7, wobei
die Druckerhöhungs- und -verringerungseinheit unmittelbar so ausgelegt ist, dass sie, bevor sich der Verbindungsabschnitt dem Gegenverbindungsabschnitt nähert und mit diesem verbunden wird, Gas zwischen der ersten Kontaktfläche des Verbindungsabschnitts und einer zweiten Kontaktfläche des Gegenverbindungsabschnitt ablässt oder einsaugt.

## Revendications

1. Dispositif de mesure de pression (1) auquel et duquel un élément de détachement-attachement (11) comportant un canal de circulation de fluide (2) dans lequel un fluide peut s'écouler est destiné à être attaché et détaché et lequel est destiné à mesurer une pression dans une cible de mesure qui est ledit élément d'attachement-détachement, ledit dispositif de mesure de pression comprenant :
un canal de circulation de communication (14C) disposé pour permettre une communication de gaz avec le canal de circulation de fluide ;
une partie de connexion (131) à laquelle une partie complémentaire de connexion (117) dudit élément d'attachement-détachement est connectée pour mettre le canal de circulation de fluide et le canal de circulation de communication dans un état de communication ou un état de non-communication ;
un capteur de pression (15, 18) destiné à mesurer une pression dans le canal de circulation de fluide et le canal de circulation de communication dans le cas où la partie complémentaire de connexion est connectée à la partie de connexion pour mettre le canal de circulation de fluide et le canal de circulation de communication dans l'état de communication ;
une unité d'augmentation-diminution de pression (17) qui est destinée à augmenter ou diminuer une pression dans le canal de circulation de communication pour décharger ou aspirer du gaz vers/de l'extérieur ;
**caractérisé par** :
une unité de commande (50) qui est destinée à causer une commande manuelle ou automatique de l'unité d'augmentation-diminution de pression à un instant où une première surface de contact de la partie de connexion à mettre en contact étanche à l'air avec la partie complémentaire de connexion est située à distance de ladite partie complémentaire de connexion,
la première surface de contact (132) de la partie de connexion étant formée à un endroit où du gaz est déchargé ou à un endroit où du gaz est aspiré par ladite unité d'augmentation-diminution de pression destinée à augmenter ou diminuer la pression dans l'état de non-communication où la partie de connexion est située à distance de ladite partie complémentaire de connexion.

2. Dispositif de mesure de pression selon la revendication 1, dans lequel
la partie de connexion est fabriqué en matériau élastique (132) de façon que la première surface de contact est apte à venir en contact étanche à l'air avec la partie complémentaire de connexion.

3. Dispositif de mesure de pression selon la revendication 1 ou 2, dans lequel
le canal de circulation de communication comprend une unité d'ouverture-fermeture (161, 162) qui est destinée à commuter ladite unité d'augmentation-diminution de pression et la cible de mesure vers un état de communication ou un état de fermeture, et
après que la pression dans le canal de circulation de communication est augmentée ou diminuée dans un état de fermeture, ladite unité d'ouverture-fermeture effectue une commutation vers l'état de communication de façon que du gaz est soufflé sur la première surface de contact de la partie de connexion.

4. Dispositif de mesure de pression selon l'une quelconque des revendications 1 à 3, dans lequel
ladite unité d'augmentation-diminution de pression est apte non seulement à effectuer un entraînement à rotation normale pour augmenter la pression dans le canal de circulation de communication, mais également un entraînement à rotation inverse pour diminuer la pression dans le canal de circulation de communication.

5. Dispositif médical (M) comprenant le dispositif de mesure de pression selon l'une quelconque des revendications 1 à 4, dans lequel
le canal de circulation de fluide est un canal de circulation de sang dans lequel du sang peut s'écouler,
ledit élément d'attachement-détachement comprend une chambre qui fait partie du canal de circulation de sang et qui est apte à retenir du sang et du gaz, et
la cible de mesure est un espace interne de la chambre.

6. Dispositif médical selon la revendication 5, comprenant en outre une unité de commande (P) pour commander l'entraînement de ladite unité d'augmentation-diminution de pression.

7. Dispositif médical selon la revendication 5 ou 6, dans lequel
ladite unité de commande est adaptée pour commencer automatiquement l'entraînement de ladite unité d'augmentation-diminution de pression dès lors qu'il est confirmé qu'un utilisateur effectuant une opération est détecté ou une opération effectuée par un utilisateur est détecté.

8. Dispositif médical selon l'une quelconque des revendications 5 à 7, dans lequel
ladite unité de commande est adaptée pour arrêter automatiquement l'entraînement de ladite unité d'augmentation-diminution de pression à un instant où une valeur mesurée par le capteur de pression atteint une pression prédéterminée préétablie.

9. Dispositif médical selon l'une quelconque des revendications 5 à 7, dans lequel
immédiatement avant que la partie de connexion ne soit approchée et ne soit reliée à la partie complémentaire de connexion, ladite unité d'augmentation-diminution de pression est adaptée à décharger ou aspirer du gaz entre la première surface de contact de la partie de connexion et une deuxième surface de contact de la partie complémentaire de connexion.
